# EUROPEAN PATENT APPLICATION

(11) **EP 4 309 655 A1**
(43) Date of publication of application: **24.01.2024**
(21) Application number: 22382706.4
(22) Date of filing: 22.07.2022
(51) Int. Cl.: A61K 31/47, A61P 27/16, A61K 9/00

(54) **QUINOLYLNITRONE DERIVATIVES FOR USE IN THE PREVENTION AND/OR TREATMENT OF HEARING LOSS**

(71) Applicant: Consejo Superior de Investigaciones Científicas (CSIC), 28006 Madrid (ES); Consorcio Centro de Investigación Biomédica en Red, 28029 Madrid (ES); Universidad Autónoma de Madrid, 28049 Madrid (ES); Fundación para la Investigación Biomédica del Hospital Universitario Ramón y Cajal, 28034 Madrid (ES)
(72) Inventor: MARCO CONTELLES, José Luis, 28006 Madrid (ES); MURILLO CUESTA, Silvia, 28029 Madrid (ES); RODRÍGUEZ DE LA ROSA, Lourdes, 28029 Madrid (ES); VARELA NIETO, Isabel, 28029 Madrid (ES); ALCÁZAR GONZÁLEZ, Alberto, 28034 Madrid (ES)
(74) Representative: Hoffmann Eitle

(57) **Abstract**

The present invention provides derivatives of quinolylnitrones of formula I, wherein the meaning of the substituents is as described in the description, useful in treatment and/or prevention of hearing loss.

Advantageously, these quinolylnitrones of formula (I) have a broad-spectrum effect, being efficient in the prevention and treatment of hearing loss regardless of its origin, whether caused by a physical agent, such as noise, or an ototoxic agent such as H₂O₂.

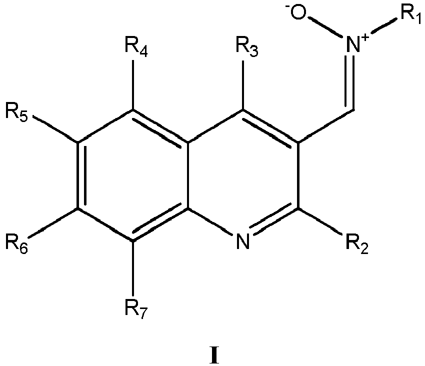

## Description

The present invention relates to the field of therapy, in particular to the prevention and/or treatment of hearing loss (hereinafter also referred as hypoacusis). In particular, the present invention relates to derivatives of quinolylnitrones of formula I, as well as to pharmaceutical compositions comprising thereof, for use in the treatment and/or prevention of hearing loss.

### BACKGROUND OF THE INVENTION

Hearing loss is an important public health problem due to its high prevalence and impact on quality of life. In children it hinders language acquisition and in adults it hinders social integration and is associated with the progression of cognitive impairment.

Hearing loss results from damage to one of the outer ear, the middle ear and the inner ear or multiple parts of them. There are four types of hypoacusis. The first most common type is sensorineural hearing loss that occurs mostly as a result of loss or damage to the auditory cells (hair cells) in the cochlea. The second type is conductive hearing loss which occurs when there is a problem with the outer or middle ear, resulting in sound not being conducted properly to the inner ear. The third type is mixed hearing loss which occurs when both sensorineural and conductive hearing losses are present. The fourth type is auditory neuropathy which occurs when there is a problem with the auditory nerve that transmits the nervous signal from the cochlea to the brain.

There are numerous environmental factors that contribute to hearing loss, most notably exposure to noise and ototoxic substances. Hearing loss caused by exposure to excessive noise is the most common hearing loss after presbycusis and is the third leading cause of sick leave. Ototoxicity is a frequent side effect associated with many drugs in clinical use, such as antibiotics, antitumor, anti-inflammatory or antimalarial drugs. Oxidative stress is a key pathological mechanism in both forms of hearing loss, producing damage to inner ear hair cells and neurons, activation of inflammatory processes and cell death by apoptosis.

The control of oxidative stress by means of antioxidant and anti-inflammatory substances constitutes a powerful therapeutic strategy to maintain homeostasis and avoid cell death.

There is currently no curative treatment for hearing loss on the market and only devices such as hearing aids or cochlear implants are available.

There is therefore the need of new pharmacological therapies which efficiently prevent or treat hearing loss.

### DESCRIPTION OF THE INVENTION

The inventors of the present invention have found that quinolylnitrones of formula (I) are efficient in the prevention and treatment of auditory cell damage and hearing loss.

Surprisingly, the inventors have found that these quinolylnitrones of formula (I) have a broad-spectrum effect, being efficient in the prevention and treatment of auditory cell damage/hearing loss regardless of its origin, whether caused by a physical agent such as noise (Example 1) or by an ototoxic agent such as H₂O₂ (Example 2).

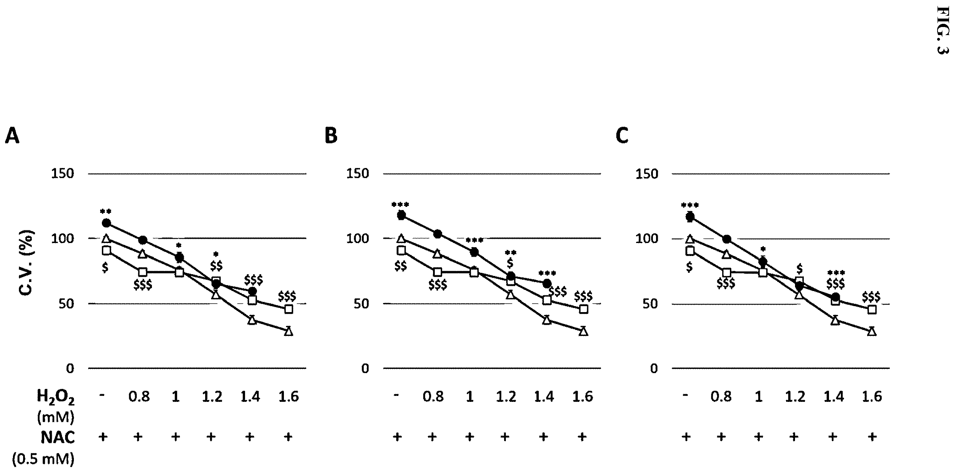

Furthermore, when the inventors compared the protective effect provided by the compounds of formula (I) towards H₂O₂ (as ototoxic agent), with the effect provided by the commercial reference product N-acetyl cysteine, they found that the compounds of formula (I) were able to provide an efficient protective effect even before administering H₂O₂. Not only that, but also that such efficiency was not negatively affected as the H₂O₂ dose was increased. The reference product, however, showed efficacy only at the highest concentrations of H₂O₂, i.e. only when the damage was most significant (FIG. 3). This is indicative that compounds of formula (I) can provide a protective effect from the earliest stages auditory damage/hearing loss. This is an improvement over NAC, which is only able to show some protective effect when damage begins to be significant.

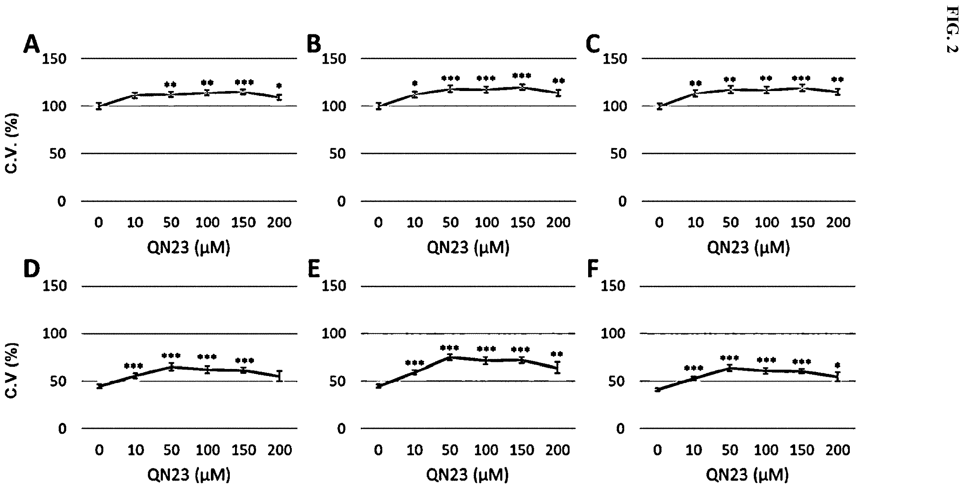

The data provided here is a major breakthrough in the amelioration of hearing loss and provides the medical expert with new safe therapeutic approaches (molecules of formula (I) do not show any toxicity as also shown in FIG. 2) that can be recommended at the slightest doubt of possible hearing loss.

Thus, the present invention provides a compound of formula (I): 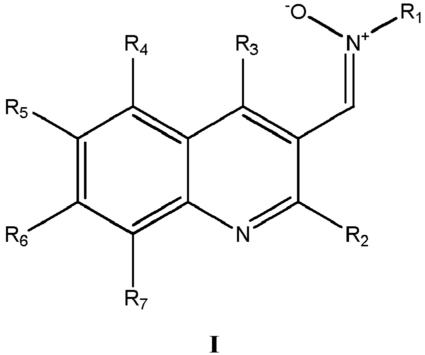 wherein:
R₁ represents a -C₁₋₅ alkyl or a known aromatic ring having 5 or 6 members selected from: -CH-, - CR₁₂-, -O-, -N-, and -S-;
each one of R₂ to R₇ independently represents -H, halogen, -OR₈ or -NHR₈;
each one R₈ independently represents -H or -C₁₋₅ alkyl, wherein the -C₁₋₅ alkyl is optionally substituted by one or more R₉;
each one R₉ independently represents, halogen, -OH, -O-C₁₋₅ alkyl, -NH₂, -NH(C₁₋₅ alkyl) or Cyi; and
Cy₁ represents a known ring system having from 5 to 8 members, saturated, partially unsaturated or aromatic, optionally containing from 1 to 3 heteroatoms selected from N, O, Se and S, Cy₁ being optionally bound to the remaining structure through any C or N atom, and optionally substituted by one or more R₁₀;
each one R₁₀ independently represents a -C₁₋₅ alkyl optionally substituted by one or more R₁₁; and each R₁₁ independently represents -C₂₋₅ alkynyl,
provided that at least two of the R₂ to R₇ are other than -H for use in a method for the prevention and/or treatment of hearing loss. This aspect can also be formulated as the use of a compound of formula (I) as defined above, for the manufacture of a medicament for the prevention and/or treatment of hearing loss. This aspect can alternatively be formulated as a method for the prevention and/or treatment of hearing loss in a subject, the method comprising the step of administering a therapeutically effective amount of the compound of formula (I) as defined above to the subject in need thereof.

### BRIEF DESCRIPTION OF THE DRAWINGS

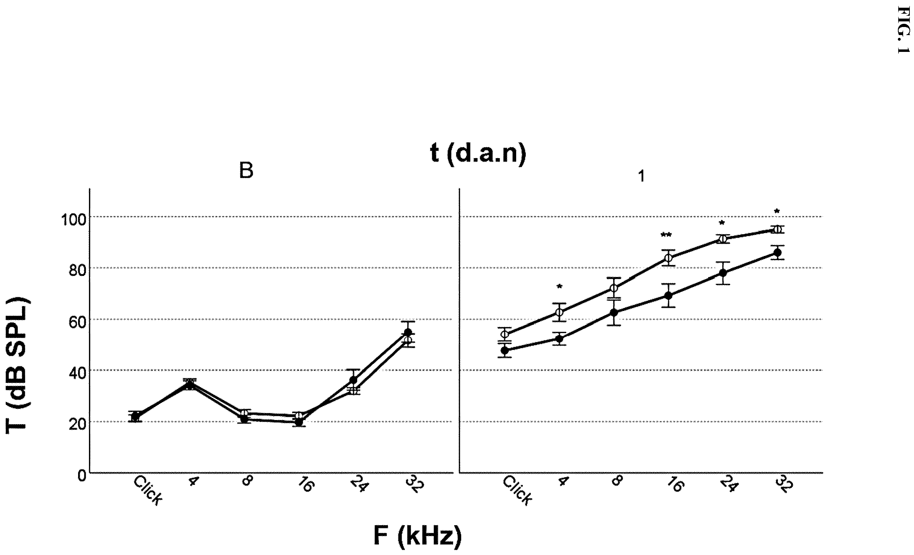

FIG. 1. Evolution of Auditory Brainstem Response thresholds (mean±SEM, in dB SPL) in response to click and pure tones (from 4 to 32 kHz) stimuli throughout the experiment in mice treated with QN23 (black circles) compared with those that received vehicle (white cercles). Results are from 3 independent experiments with an n of at least 6 mice per group each. Statistical significance was calculated by Mann-Whitney U test, ^{∗}p<0.05; ^{∗∗}p<0.01 in comparison vs. control not treated with QN23. T= threshold; F= frequency, t (d.a.n.)= time (day after noise); B= baseline.
FIG. 2. Viability assay in HEI-OC1 cells treated with different doses of QN23 with and without H₂O₂ for 2h. Data are represented as mean ± SEM. Results are from 3 independent experiments and 7 wells/condition/experiment for QN23 nitrone without H₂O₂ treatment (A-C) or with QN23 treatment (D-E). Statistical significance was calculated by Student's test: ^{∗}p<0.05; ^{∗∗}p<0.01; ^{∗∗∗}p<0.001 in comparison vs. untreated control with QN23.
FIG. 3. Viability assay in HEI-OC1 cells treated with 50 µM QN23 (circle), 0.5 mM NAC (square) and increasing doses of H₂O₂ (0.8-1.6 mM) (triangle) for 2 h. After this time, the treatment was removed and fresh medium and XTT reagents were added. Cell viability was measured by XTT assay. Results are from 3 independent experiments. Statistical significance was calculated by Student's test: ^{∗}QN23 vs H₂O₂ (^{∗}<0,05; ^{∗∗}p<0,01; ^{∗∗∗}p<0,001). ^{$}NAC vs H₂O₂ (^{$}p<0,05; ^{$$}p<0,01; ^{$$$}p<0,001).

### DETAILED DESCRIPTION OF THE INVENTION

Throughout the present description, the term "treatment" refers to eliminating, reducing or lessening the cause or effects of a disease or disorder. For the purposes of this invention, treatment includes, but is not limited to, alleviating, diminishing or eliminating one or more symptoms of the disorder (i.e. hearing loss); reducing the degree of the loss, stabilizing (i.e., not worsening) the condition, delaying or slowing the progression of the hearing loss, alleviating or improving its condition, and remitting (whether total or partial).

As used in the present invention, the term "prevention" refers to preventing the onset of the disease from occurring in a patient who is predisposed to or has risk factors, but who does not yet have symptoms of the disease. Prevention also includes preventing the recurrence of a disease in a subject who has previously suffered from that disease.

As used herein, the term "therapeutically effective amount" means the amount of a drug that will elicit the biological or medical response in a tissue, system, animal or human being that is sought, for example, by an investigator or clinician. In addition, the term "therapeutically effective amount" means any amount that, in comparison to a corresponding subject who has not received such amount, results in an improvement in the treatment, cure, prevention or amelioration of a disease, disorder or side effect, or a decrease in the rate of progression of a disease or disorder. The term also includes within its scope amounts effective in improving normal physiological function.

In the present invention, the term "alkyl" is to be understood as a straight or branched acyclic saturated chain alkyl substituent. Illustrative non-limiting examples are methyl, ethyl, propyl, butyl, 1-methylethyl, 1-methylpropyl, 2-methylpropyl or 1,1-dimethylethyl.

In the present invention, the term "halogen" means fluorine, chlorine, bromine or iodine.

In one embodiment the compound of formula I one wherein:
R₁ represents a -C₁₋₅ alkyl or a known aromatic ring having 5 or 6 members selected from: -CH-, - CR₁₂-, -O-, -N-, and -S-;
each one of R₂ to R₇ independently represents -H, halogen, -OR₈ or -NHR₈; and each one R₈ independently represents -H or -C₁₋₅ alkyl.

In another embodiment the compound of formula I is one wherein R₁ represents C₁₋₅ alkyl, such as methyl, ethyl or tert-butyl.

In another embodiment the compound of formula I is one wherein each one R₂ to R₇ independently represents-H, halogen or -OR₈.

In another embodiment the compound of formula I is one wherein:
R₁ represents C₁₋₅ alkyl; and
each one R₂ to R₇ independently represents-H, halogen or -OR₈.

In one embodiment the compound of formula (I) is one wherein:
R₁ represents -C₁₋₅ alkyl;
each one R₂ to R₇ independently represents-H, halogen, or -OR₈; and
R₈ independently represents-H or-C₁₋₅ alkyl;

In another embodiment the compound of formula I is one wherein R₃, R₄, R₆ and R₇ represent -H.

In another embodiment the compound of formula I is one wherein:
R₁ represents C₁₋₅ alkyl; and
R₃, R₄, R₆ and R₇ represent -H.

In another embodiment the compound of formula I is one wherein R₈ represents -OR₈.

In another embodiment the compound of formula I is one wherein:
R₁ represents C₁₋₅ alkyl;
R₈ represents -OR₈; and
R₃, R₄, R₆ and R₇ represent -H.

In another embodiment the compound of formula I is one wherein R₂ represents halogen.

In another embodiment the compound of formula I is one wherein:
R₂ represents halogen; and
R₈ represents -OR₈.

In another embodiment the compound of formula I is one wherein:
R₁ represents C₁₋₅ alkyl, such as *tert*-butyl; and
R₂ represents halogen.

In another embodiment the compound of formula I is one wherein:
R₁ represents C₁₋₅ alkyl, such as *tert*-butyl;
R₂ represents halogen; and
R₃, R₄, R₆ and R₇ represent -H.

In another embodiment the compound of formula I is one wherein:
R₁ represents C₁₋₅ alkyl, such as *tert*-butyl;
R₂ represents halogen;
R₈ represents -OR₈; and
R₃, R₄, R₆ and R₇ represents -H.

In another embodiment the compound of formula I is one wherein R₂ represents chlorine.

In another embodiment the compound of formula I is one wherein R₈ represents -C₁₋₅ alkyl. In another embodiment the compound of formula I is one wherein R₈ represents methyl.

In another embodiment the compound of formula (I) is (Z)-N-*tert*-butyl-1-(2-chloro-6-methoxyquinolin-3-yl)methanimine oxide (hereinafter also referred as "QN23").

The compounds of the present invention can be in the form of a pure optical isomer, or a mixture of optical isomers in all proportions, or in a form enriched in an optical isomer, and their pharmaceutically acceptable salts, solvates or hydrates.

Solvates of the compounds of the present invention are also contemplated herein. "Solvate" means a physical association of a compound of this invention with one or more solvent molecules. This physical association involves varying degrees of ionic and covalent bonding, including hydrogen bonding. In certain instances, the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is H₂O.

The compounds represented by structural formula I may form salts which are also within the scope of this invention. Reference to a compound represented by structural formula I herein is understood to include reference to salts thereof, particularly pharmaceutically acceptable salts thereof, unless otherwise indicated. The term "salt(s)", as employed herein, denotes acidic salts formed with inorganic and/or organic acids, as well as basic salts formed with inorganic and/or organic bases. In addition, when a compound represented by structural formula I contains both a basic moiety, such as, but not limited to, a pyridine or imidazole, and an acidic moiety, such as, but not limited to a carboxylic acid, zwitterions ("inner salts") may be formed and are included within the term "salt(s)" as used herein. Pharmaceutically acceptable salts are preferred, although other salts are also useful. Salts of the compounds represented by structural formula I may be formed, for example, by reacting such as compound with an amount of acid or base, such as an equivalent amount, in a medium such as one in which the salt precipitates or in an aqueous medium followed by lyophilization.

Exemplary acid addition salts include acetates, adipates, alginates, ascorbates, aspartates, benzoates, benzenesulforiates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, 2-hydroxyethanesulfonates, lactates, maleates, methanesulfonates, 2-naphthalenesulfonates, nicotinates, nitrates, oxalates, pectinates, persulfates, 3-phenylpropionates, phosphates, picrates, pivalates, propionates, salicylates, succinates, sulfates, sulfonates, tartarates, thiocyanates, toluenesulfonates (also known as tosylates) undecanoates, and the like.

Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, hydrabamines (formed with N,N-bis(dehydroabietyl)ethylenediamine), N-methyl-D-glucamines, N-methyl-D-glucamides, t-butyl amines, and salts with amino acids such as arginine, lysine and the like. Basic nitrogen-containing groups may be quartemized with agents such as lower alkyl halides (e.g. methyl, ethyl, propyl, and butyl chlorides, bromides and iodides), dialkyl sulfates (e.g. dimethyl, diethyl, dibutyl, and diamyl sulfates), long chain halides (e.g. decyl, lauryl, myristyl and stearyl chlorides, bromides and iodides), aralkyl halides (e.g. benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Compounds represented by structural formula I, and salts and solvates thereof, may exist in their tautomeric form (for example, as an amide or imino ether). All such tautomeric forms are contemplated herein as part of the present invention.

The compounds of formula I of the present invention and their salts and solvates as well as isomers are toxicologically safe and, accordingly, suitable as pharmacologically active ingredients in pharmaceutical compositions.

Thus, in one embodiment, the compound of formula (I) is administered in the form of a pharmaceutical composition comprising, in addition, at least one pharmaceutically acceptable adjuvant, excipient and/or vehicle and/or, if appropriate, one or more additional pharmacologically active compounds.

For application in therapy, the compounds of formula I, salts or isomers thereof will preferably be in a pharmaceutically acceptable or substantially pure form, i.e., with a pharmaceutically acceptable level of purity excluding normal pharmaceutical additives such as diluents or carriers, or not including material considered toxic at normal dosage levels. The purity levels for the active principle are preferably greater than 50%, more preferably greater than 70%, or still more preferably greater than 90%. In a preferred embodiment, they are greater than 95% of compound of formula I.

The amount of the compound to be administered to patients is variable and depends for example on the weight or age of the patient or also on the type of administration, the indication or the severity of the disease. Typically, between 0.001 or 100 mg/kg body weight of the patient is administered.

The pharmaceutical compositions can be prepared as a liquid, semi-solid or solid dosage form, for example in the form of solutions for injection, drops, juices, syrups, sprays, suspensions, tablets, patches, capsules, dressings, suppositories, ointments, creams, lotions, gels, emulsions, aerosols or in multiparticulate form, for example in the form of pills or granules, if appropriate compressed into tablets, decanted into capsules or suspended in a liquid, or administered as such.

These compositions can be prepared with the aid of conventional means, devices, methods or processes known in the art.

Pharmaceutically acceptable adjuvants, vehicles or excipients which may be used in such compositions are adjuvants, vehicles or excipients known to those skilled in the art or commonly used in the preparation of therapeutic compositions, which may be selected, for example, from the group consisting of excipients, fillers, solvents, diluents, surfactants, colorants, preservatives, disintegrants, sliding agents, lubricants, flavouring agents or binders.

The term "pharmaceutically acceptable" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans without excessive toxicity, irritation, allergic response, immunogenicity or other problems or complications commensurate with a reasonable benefit/risk ratio.

The selection of physiologically compatible adjuvants or the number of adjuvants to be used depends on the form of administration of the pharmaceutical composition, i.e. oral, subcutaneous, parenteral, intravenous, intraperitoneal, intradermal, intramuscular, intranasal, buccal, rectal or intratympanic. Preparations in the form of tablets, dragees, capsules, granules, pills, drops, particularly otic drops, juices or syrups are preferably suitable for oral administration; solutions, suspensions, easily reconstitutable dry preparations or also sprays are preferably suitable for parenteral, topical or inhalation administration. The compounds in accordance with the invention used in the pharmaceutical composition in accordance with the invention in a depot, in a dissolved form or in a dressing, or if appropriate having added other agents favoring penetration into the skin, are preparations suitable for percutaneous administration. The preparation forms administrable orally or percutaneously can also release the respective compound according to the invention in a delayed form.

In one embodiment the pharmaceutical composition is in a solid form or in aqueous suspension, together with a pharmaceutically acceptable diluent, for oral, topical, intratympanic, or parenteral administration.

In another embodiment the pharmaceutical composition is suitable for parenteral administration, particularly suitable for subcutaneous, intraperitoneal, intradermal, intramuscular, or intravenous administration.

In another embodiment the hearing loss is due to a physical agent, such as noise.

In another embodiment the hearing loss is due to an exposure to an ototoxic agent.

In the context of the invention an "ototoxic agent" is a substance that through its chemical or mechanical action, injures, impairs, or inhibits the activity of a component of the nervous system related to hearing, thereby resulting in impair hearing. The list of ototoxic agents that cause hearing impairments includes, but is not limited to, antineoplastic agents such as vincristine, vinblastine, cisplatin, taxol, or dideoxy-compounds, e.g., dideoxyinosine; alcohol; metals; industrial toxins involved in occupational or environmental exposure (like toluene); contaminants of food or medicinals; or over-doses of vitamins or therapeutic drugs, e.g., antibiotics such as penicillin or chloramphenicol, or megadoses of vitamins A, D, or B6, salicylates, quinines and loop diuretics. Loud noise is also meant to be included in the list of ototoxic agents. By "exposure to an ototoxic agent" is meant that the ototoxic agent is made available to, or comes into contact with a mammal. Exposure to an ototoxic agent can occur by direct administration, e.g., by ingestion or administration of a food, medicinal, or therapeutic agent, e.g., a chemotherapeutic agent, by accidental contamination, or by environmental exposure, e.g., aerial or aqueous exposure. Foetal exposure may also occur systemically when a drug given the mother crosses the placental barrier.

The compounds of formula (I) and pharmaceutical compositions can be administered before the exposition to the physical or ototoxic agent; or alternatively after the exposition to the physical or ototoxic agent; or alternatively both before and after the exposition to the physical or ototoxic agent.

In another embodiment the compounds of formula (I) and pharmaceutical compositions can be administered once, twice or three times a day.

Throughout the description and claims the word "comprises" or variants thereof are not intended to exclude other technical features, additives, components or steps. To those skilled in the art, other objects, advantages or features of the invention will be apparent in part from the description or in part from the practice of the invention. The following examples are provided by way of illustration, or are not intended to be limiting of the present invention.

### EXAMPLES

The following table lists the different tests carried out with QN23 nitrone for the treatment of hypoacusis induced by noise exposure in mice.

The experiments were carried out in C57BL/6JCrl mice, male or female, aged 1.5 months at the beginning of the study.

Noise exposure was performed inside a reverberation chamber. The animals were exposed to 105 dB SL for 30 minutes (Sanz L, Murillo-Cuesta S, Cobo P, Cediel-Algovia R, Contreras J, Rivera T, Varela-Nieto I, Avendaño C. Swept-sine noise-induced damage as a hearing loss model for preclinical assays. Front Aging Neurosci. 2015 Feb 16;7:7).

Auditory function was assessed by recording auditory brainstem evoked potentials (ABR) at baseline and after noise exposure (1 day, 14 days and 28 days after exposure), according to protocol described in (Murillo-Cuesta S, Contreras J, Zurita E, Cediel R, Cantero M, Varela-Nieto I, Montoliu L. Melanin precursors prevent premature age-related and noise-induced hearing loss in albino mice. Pigment Cell Melanoma Res. 2010 Feb;23(1):72-83. doi: 10.1111/j.1755-148X.2009.00646.x. Epub 2009 Oct 19).

The nitrone QN23 was prepared as described in WO2020/074666. The solution for its administration was always performed at the time of use by dissolving it under vigorous stirring in a mixture of ethanol, polyethylene glycol (PEG400, Sigma) and saline (1:200:60, V/V), until a stock solution of concentration 3.6 mg/ml was obtained. The stock solution was then diluted in a mixture of polyethylene glycol (PEG400, Sigma) and saline (6:7, V/V) to a final concentration of 0.25 mg/ml (854 µM). The nitrone solution was injected intraperitoneally during the two hours following its preparation, discarding the unused remains.

Treatments with QN23 nitrone were always started one hour before exposure to noise and continued for 3 days, according to the dosage of 2 mg/kg/12 h.

FIG. 1 shows the results obtained with QN23 nitrone administered at 2 mg/kg every 12 h for 3 consecutive days. Before noise exposure, all animals showed a similar audiometric profile. At 24 h after noise exposure, an increase in thresholds in response to all frequencies was observed, with QN23-treated animals having significantly lower thresholds than those receiving vehicle. Indicative of a noise-protective effect.

### Study of the otoprotective role of QN23 in cell cultures.

QN23 nitrone was tested in cell cultures of HEI-OC1 mouse auditory cells derived from the Immortomouse^{™} postnatal organ of Corti following protocols published by the group (Cervantes et al., J Clin Med. 2019 Sep 14;8(9):1464). HEI-OC1 cells were cultured in DMEM medium supplemented with 10% fetal bovine serum (Gibco), without antibiotics.

Cells were treated with different doses of QN23 (10-50-100-150-200 µM) and with or without 1.2 mM H₂O₂. Cell viability was studied by XTT assay. Absorbance measurements at 450 and 660 nm were taken with the VERSAmax plate reader at 4 h, 6 h and 23 h after addition of XTT reagents. Viability results were calculated following the formula: Specific absorbance = [Abs450 nm (test) - Abs450 nm (blank)] - Abs660 nm (test). The wells without cells in the respective treatments were used as blanks. Data are represented as mean ± SEM, considering the untreated control as 100% viability. Results are from 3 independent experiments and 7 wells/condition/experiment for QN23 nitrone without H₂O₂ treatment (A-C) or with treatment (D-E). Statistical significance was calculated by Student's test: ^{∗}p<0.05; ^{∗∗}p<0.01; ^{∗∗∗}p<0.001 in comparison vs. untreated control with QN23. The results are provided in FIG. 2. Figures 2A to 2C show that the administration and treatment for 2 hours with QN23, prior to administering H₂O₂, was not toxic to the cells. After the exposure to H₂O₂, the cell viability was reduced about 50% due to the cell death associated to the oxidative damage caused by H₂O₂. QN23 treatment significantly increased cell viability at all the doses tested with respect to the non-treated control group, specially at high dose of 1.2 mM (Figure 2D-2F).

The inventors also compared the effect of QN23 with N-acetylcysteine (NAC). To that end, QN23 nitrone was dissolved in absolute EtOH ≥ 99.5% (#107017; Merck) and tested *in vitro* in a dose range of 10-200 µM. Cells were treated with increasing doses of H₂O₂ (1-1.4 mM; Merck).

N-acetylcysteine (NAC) (0.5 mM; Sigma) was used as a reference antioxidant treatment. NAC is a prodrug of L-cysteine, itself a precursor of the biological antioxidant glutathione, among other intracellular actions (Ezerina et al., Cell Chem Biol. 2018 Apr 19;25(4):447-459.e4). Cells were treated with increasing doses of H₂O₂ (0.8-1.6 mM).

In both cases, survival curves were performed using the CyQUANT^{™} XTT cell viability assay (#X12223; Invitrogen) after 2 hours of the administration of QN23 or NAC. The results are summarized in FIG. 3.

The results of experiments with NAC showed that the compound effectively protected from H₂O₂-induced oxidative damage and cell death at the highest doses tested, contrary to QN23, which provides an efficient protective effect even before H₂O₂ administration. This protective effect makes cell viability superior at all H₂O₂ concentrations tested.

From this FIG. 3, another advantage can also be derived: QN23 is sufficiently "sensitive" to provide a protective effect even in the early stages of cell deterioration. As can be seen, QN23 provides an enhancement of cell viability even at concentrations as low as 0.8 mM H₂O₂. This protective effect is not provided by NAC, which shows to be efficient in the protection/recovery of cell viability only in cases where H₂O₂ is administered at higher concentrations, i.e., once the damage is late stages without the recovery of cell viability being significantly higher.

The protective effect provided by QN23 in the earliest stages of the lesion together with the absence of toxicity may be of great importance for the physician who could efficiently manage this type of hearing impairment from the first symptoms or suspicions of a possible hearing loss.

## Claims

1. A compound of formula **I**: 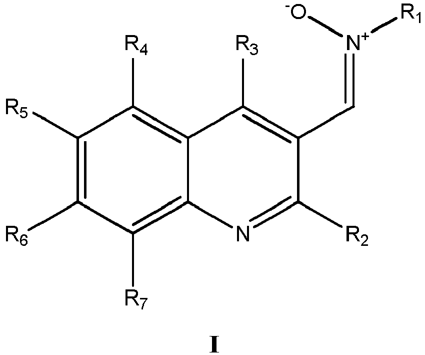 wherein:
R₁ represents a -C₁₋₅ alkyl or a known aromatic ring having 5 or 6 members selected from: -CH-, - CR₁₂-, -O-, -N-, and -S-;
each one of R₂ to R₇ independently represents -H, halogen, -OR₈ or -NHR₈;
each one R₈ independently represents -H or -C₁₋₅ alkyl, wherein the -C₁₋₅ alkyl is optionally substituted with one or more R₉;
each one R₉ independently represents halogen, -OH, -O-C₁₋₅ alkyl, -NH₂, -NH(C₁₋₅ alkyl) or Cyi; and Cy₁ represents a known ring system having from 5 to 8 members, saturated, partially unsaturated or aromatic, optionally containing from 1 to 3 heteroatoms selected from N, O, Se and S, Cy₁ being optionally bound to the remaining structure through any C or N atom, and optionally substituted by one or more R₁₀;
each one R₁₀ independently represents a -C₁₋₅ alkyl optionally substituted with one or more R₁₁; and each R₁₁ independently represents -C₂₋₅ alkynyl ,provided that at least two of the R₂ to R₇ are other than -H;
for use in a method for the prevention and/or treatment of hearing loss.

2. The compound for use according to claim 1, wherein:
R₁ represents a -C₁₋₅ alkyl or a known aromatic ring having 5 or 6 members selected from: -CH-, - CR₁₂-, -O-, -N-, and -S-;
each one of R₂ to R₇ independently represents -H, halogen, -OR₈ or -NHR₈; and
each one R₈ independently represents -H or -C₁₋₅ alkyl.

3. The compound of formula (I) for use according to any one the preceding claims, wherein R₁ represents C₁₋₅ alkyl.

4. The compound of formula (I) for use according to any one of the preceding claims, wherein R₁ represents tert-butyl.

5. The compound of formula (I) for use according to any one of the preceding claims, wherein each one R₂ a R₇ independently represents-H, halogen or -OR₈.

6. The compound of formula (I) for use according to any one of the preceding claims, wherein R₃, R₄, R₆ and R₇ represent -H.

7. The compound of formula (I) for use according to any one of the preceding claims, wherein R₂ represents halogen.

8. The compound of formula (I) for use according to any one of the preceding claims, wherein R₂ represents chlorine.

9. The compound of formula (I) for use according to any one of the preceding claims, wherein R₅ -OR₈.

10. The compound of formula (I) for use according to any of the preceding claims, wherein R₈ represents -C₁₋₅ alkyl.

11. The compound of formula (I) for use according to any of the preceding claims, which is (Z)-N-*tert*-butyl-1-(2-chloro-6-metoxiquinolin-3-yl)methanimine oxide (QN23).

12. The compound of formula (I) for use according to any of the preceding claims, wherein the hearing loss is due to a physical agent, such as noise.

13. The compound of formula (I) for use according to any of the preceding claims, wherein the hearing loss is due to exposure to an ototoxic agent.

14. The compound of formula (I) for use according to any of the preceding claims, wherein the method of preventing or treating comprises administering the compound before the exposition to the physical or ototoxic agent; or alternatively the compound is administered after the exposition to the physical or ototoxic agent; or alternatively the compound is administered both before and after the exposition to the physical or ototoxic agent.

15. The compound of formula (I) for use according to any of the preceding claims, which is administered in the form of a pharmaceutical composition which comprises at least one pharmaceutically acceptable adjuvant, excipient and/or vehicle; particularly in the form of an oral, topical, intratympanic, or parenteral administration; particularly in the form of otic drops.
